# EUROPEAN PATENT SPECIFICATION

(11) **EP 2 623 063 B1**
(45) Date of publication and mention of the grant of the patent: **16.09.2015**
(21) Application number: 11844452.0
(22) Date of filing: 28.11.2011
(51) Int. Cl.: A61B 19/00, B25J 19/06

(54) **SURGICAL INSTRUMENT AND SURGERY SUPPORT SYSTEM HAVING SAID SURGICAL INSTRUMENT**
CHIRURGISCHES INSTRUMENT UND CHIRURGISCHES UNTERSTÜTZUNGSSYSTEM MIT BESAGTEM CHIRURGISCHEN INSTRUMENT
INSTRUMENT CHIRURGICAL ET SYSTÈME DE SUPPORT CHIRURGICAL MUNI DUDIT INSTRUMENT CHIRURGICAL

(30) Priority: 02.12.2010 JP 2010269301
(43) Date of publication of application: 07.08.2013
(73) Proprietor: OLYMPUS CORPORATION, Shibuya-ku Tokyo 151-0072 (JP)
(72) Inventor: KISHI, Kosuke, Hachioji-shi Tokyo 192-8512 (JP)
(74) Representative: Gunzelmann, Rainer
(86) International application number: PCT/JP2011/077302
(87) International publication number: WO 2012/073849

(56) References cited:
- EP-A1- 1 038 504
- JP-A- 2006 155 199
- JP-A- 2009 153 989
- JP-A- 2010 214 128
- JP-A- 2010 227 331
- US-A- 5 359 993
- US-A1- 2004 267 297
- US-A1- 2007 055 219
- US-A1- 2009 248 043

## Description

### Technical Field

The present invention relates to an operation support system having a surgical instrument.

### Background Art

Recently, in order to reduce manpower in medical facilities, medical procedures using robots have been under study. Particularly in the field of surgery, various suggestions have been made regarding medical manipulator systems (operation support systems) that use a multidegree-of-freedom manipulator having a multidegree-of-freedom arm to treat a patient. In such a medical manipulator system, various surgical instruments such as a gripper and forceps are attached to the end of the arm. These surgical instruments have heretofore been disposed of. Recently, it has become possible to attach the same surgical instrument for use more than once by giving the surgical instrument a treatment such as sterilization. However, such a surgical instrument deteriorates after used more than once, and the deterioration can cause trouble during use. In order to prevent the trouble, it is necessary to correctly know the limit of the use (life) of the surgical instrument accordingly.

For example, Jpn. Pat. Appln. KOKAI Publication No. 2000-107189 has suggested a technique for using a surgical instrument within its life. In a configuration according to Jpn. Pat. Appln. KOKAI Publication No. 2000-107189, the surgical instrument has an internal battery so that the internal battery discharges electricity while the surgical instrument is attached to an arm. In such a configuration, according to Jpn. Pat. Appln. KOKAI Publication No. 2000-107189, the electromotive force of the internal battery is measured to measure, as the life of the surgical instrument, the time in which the surgical instrument is attached to the arm. When the electromotive force of the internal battery is equal to or less than a predetermined value, an alarm for encouraging the replacement of the surgical instrument is given.

EP 1 038 504 A1 discloses a medical apparatus comprising an apparatus body and a terminal instrument which operates when receiving a terminal output which is an electric or electromagnetic output. The terminal instrument is provided. with an installed cell of predetermined capacity and the apparatus body is provided with means for measuring the electromotive force of the installed cell while discharging it via a predetermined resistor while the terminal instrument is used, means for comparing it with a standard voltage, means for blocking the output command from the primary control section when the electromotive force of the installed cell is below the standard voltage, and means for generating an alarm when the electromotive force of the installed cell is below the standard voltage.

US 2009248043, on which the preamble of claim 1 is based, discloses a system for robotically assisted surgery with a surgical tool having its tool life data stored in a memory of the tool.

### Disclosure of Invention

When the electromotive force of an internal battery, such as that described in Jpn. Pat. Appln. KOKAI Publication No. 2000-107189, is measured, there may be a case where the actual attachment time does not correspond to the electromotive force of the internal battery, due to the natural electrical discharge of the internal battery or for some reason. If the actual attachment time of the surgical instrument does not correspond to the electromotive force, an alarm may be given before the life the surgical instrument expires. This does not enable efficient replacement of the surgical instrument.

The present invention has been made in consideration of the above circumstances, and is intended to provide an operation support system having a surgical instrument.

According to the present invention, there is provided an operation support system comprising the features of claim 1.

### Brief Description of Drawings

FIG. 1 is a diagram showing the overall configuration of a master-slave manipulator as an example of an operation support system according to embodiments of the present invention;
FIG. 2A is a first view showing the configuration of a slave arm according to the first embodiment of the present invention;
FIG. 2B is a second view showing the configuration of the slave arm according to the first embodiment of the present invention;
FIG. 3 is a flowchart showing the operation of a timer;
FIG. 4 is a diagram showing an example of the operation of a lock mechanism;
FIG. 5 is a flowchart showing the operation of a manipulator control unit;
FIG. 6 is a diagram showing the configuration of a slave arm according to the second embodiment of the present invention;
FIG. 7 is a flowchart showing the operation of a counter IC;
FIG. 8 is a diagram showing an example of the application of the configuration according to the second embodiment in which a driving mechanism of a positioning arm has a translation mechanism;
FIG. 9 is a diagram showing an example of the application of the configuration according to the first embodiment in which a distal movable portion comprises multiple movable portions;
FIG. 10A is a first view showing a configuration according to a modification in which the attachment of a surgical instrument to a positioning arm is mechanically detected to lock the operation of the surgical instrument; and
FIG. 10B is a second view showing the configuration according to the modification in which the attachment of the surgical instrument to the positioning arm is mechanically detected to lock the operation of the surgical instrument.

### Mode for Carrying Out the Invention

Embodiments according to the present invention will hereinafter be described with reference to the drawings.

### [First Embodiment]

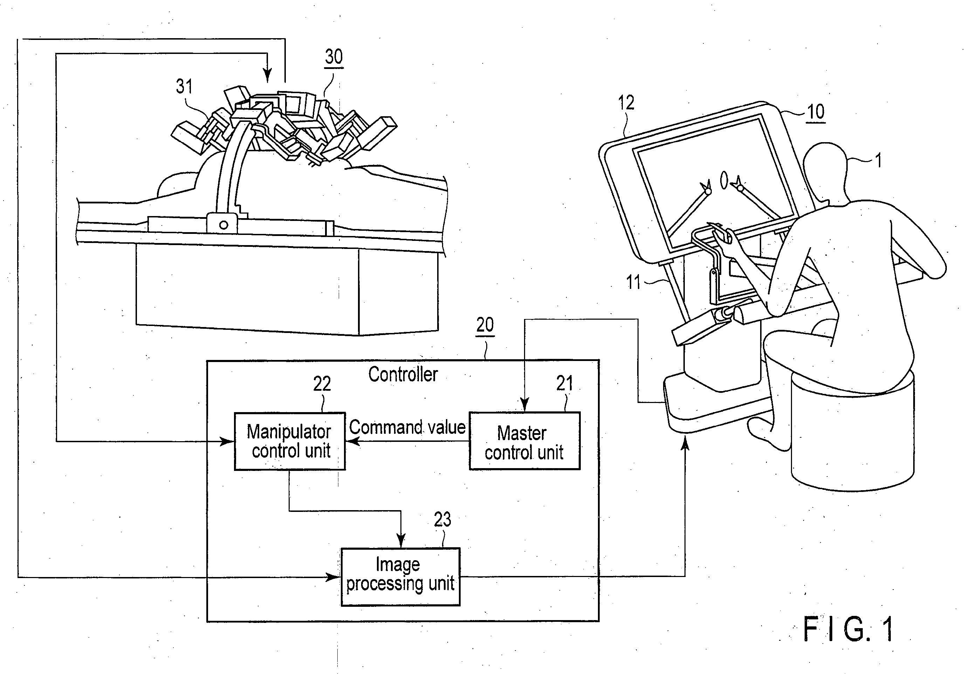

FIG. 1 is a diagram showing the overall configuration of a master-slave manipulator as an example of an operation support system according to embodiments of the present invention. As shown in FIG. 1, the master-slave manipulator according to the present embodiments comprises a remote operation device 10, a controller 20, and a slave manipulator 30.

The remote operation device 10 functions as a master in the present master-slave manipulator, and comprises an operation unit 11 and a display unit 12.

The operation unit 11 comprises, for example, driving shafts and a gripper. An operator 1 operates the operation unit 11 so that the driving shafts constituting the operation unit 11 are driven. The driving amount of each driving shaft is detected by an unshown position sensor (e.g., an encoder) provided in each driving shaft. A detection signal of each position sensor is output to the controller 20 as a signal (operation signal) that indicates operation information for the operation unit 11 to give a command regarding the position and orientation of the end of a slave arm 31 of the slave manipulator 30.

The display unit 12 comprises, for example, a liquid crystal display, and displays an image in accordance with an image signal input from the controller 20. As will be described later, the image signal input from the controller 20 is provided by processing, in the controller 20, an image signal which is obtained via an electronic camera (electronic endoscope) attached to the slave arm 31. The image based on such an image signal is displayed on the display unit 12 so that the operator 1 of the remote operation device 10 can check an image of the end of the slave manipulator 30 located apart from the remote operation device 10.

The controller 20 comprises a master control unit 21, a manipulator control unit 22, and an image processing unit 23.

The master control unit 21 calculates command values for the position and orientation of the end of the slave arm 31 in accordance with the operation signals from the remote operation device 10, and outputs the command values for the position and orientation to the manipulator control unit 22 together with a detection value detected in a later-described driving amount detecting unit.

In response to the command values for the position and orientation from the remote operation device 10, the manipulator control unit 22 calculates, for example, by inverse-kinematic computation, a desired driving amount of each joint of the slave arm 31 necessary for the position and orientation of the end of the slave arm 31 to correspond to the command values.

The image processing unit 23 processes the image signal obtained from the electronic camera (e.g., electronic endoscope) provided at the end of the slave arm 31, and generates an image signal to be displayed on the display unit 12, and then outputs the image signal to the display unit 12.

The slave manipulator 30 has the slave arm 31. The slave arm 31 comprises a positioning arm and a surgical instrument. The positioning arm has multiple joints, and is configured so that each joint is driven in accordance with a control signal from the manipulator control unit 22. The surgical instrument comprises a surgical instrument distal end, and a driving unit for driving the surgical instrument distal end. The surgical instrument distal end is attached to the distal end of the positioning arm, and the driving unit is provided in the positioning arm. A gripper, for example, is used as the surgical instrument. A camera (electronic endoscope), for example, may be attached to the distal end.

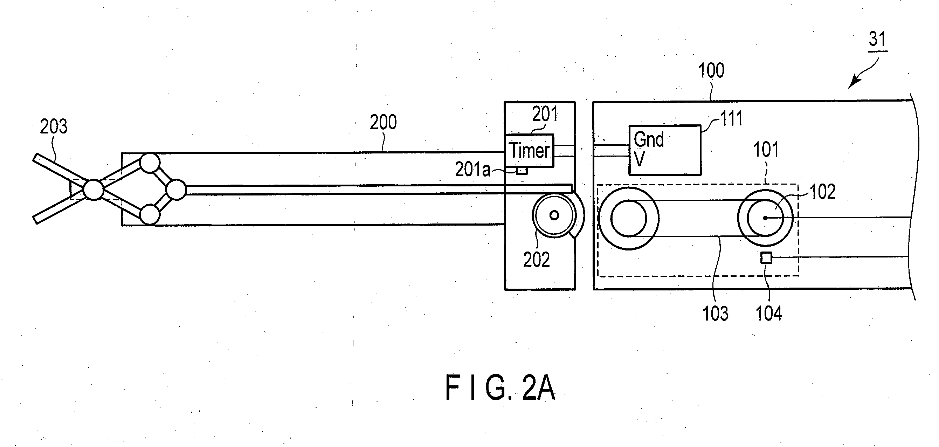

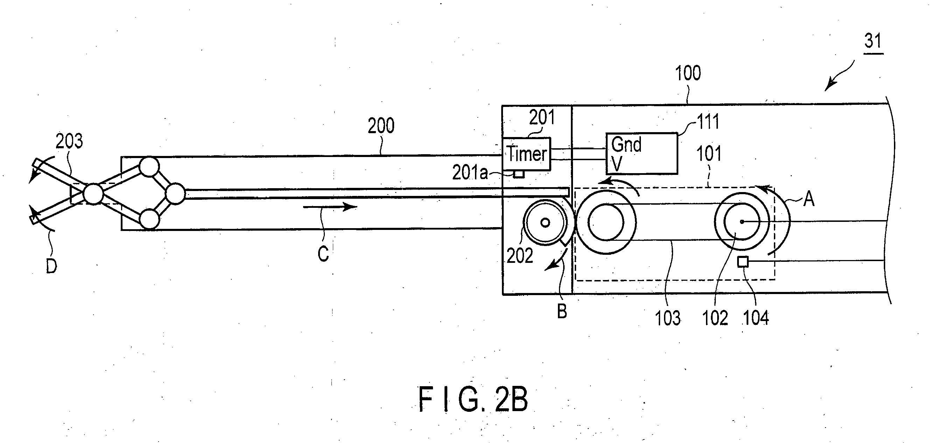

The slave arm 31 according to the present embodiments is further described with reference to FIG. 2A and FIG. 2B. As shown in FIG. 2A and FIG. 2B, the slave arm 31 comprises a positioning arm 100 and a surgical instrument distal end 200.

An attachment portion for attaching the surgical instrument distal end 200 is formed at the distal end of the positioning arm 100. The attachment portion is provided with a driving mechanism 101 which is a driving unit for driving the surgical instrument distal end 200, and an electricity supply unit 111 for supplying electricity to a timer 201 provided in the surgical instrument distal end 200.

The driving mechanism 101 comprises an actuator unit 102, a power transmission unit 103, and a driving amount detecting unit 104. The actuator unit 102 is a mechanism for generating input to drive the surgical instrument distal end 200, and comprises, for example, a motor. The actuator unit 102 generates input to drive the surgical instrument distal end 200 in accordance with a control signal from the manipulator control unit 22. The power transmission unit 103 is a mechanism for transmitting the input generated by, for example, the motor to the surgical instrument distal end 200. The power transmission unit 103 illustrated in FIG. 2A is a mechanism that uses gears and a belt to transmit the rotating input generated in the actuator unit 102 to the surgical instrument distal end 200. The configurations of the actuator unit 102 and the power transmission unit 103 shown in FIG. 2A are illustrative only, and can be suitably modified. The driving amount detecting unit 104 comprises, for example, an encoder, and detects the driving amount (rotation amount in the example of FIG. 2A) of the actuator unit 102 as an electric signal.

The electricity supply unit 111 is conductively connected to the timer 201 in the surgical instrument distal end 200 when the surgical instrument distal end 200 is attached to the positioning arm 100. The electricity supply unit 111 is connected to the timer 201, for example, by a connector. The electricity supply unit 111 having such a configuration supplies electricity for driving the timer 201.

The surgical instrument distal end 200 comprises the timer 201, a power transmission unit 202, and a distal movable portion 203.

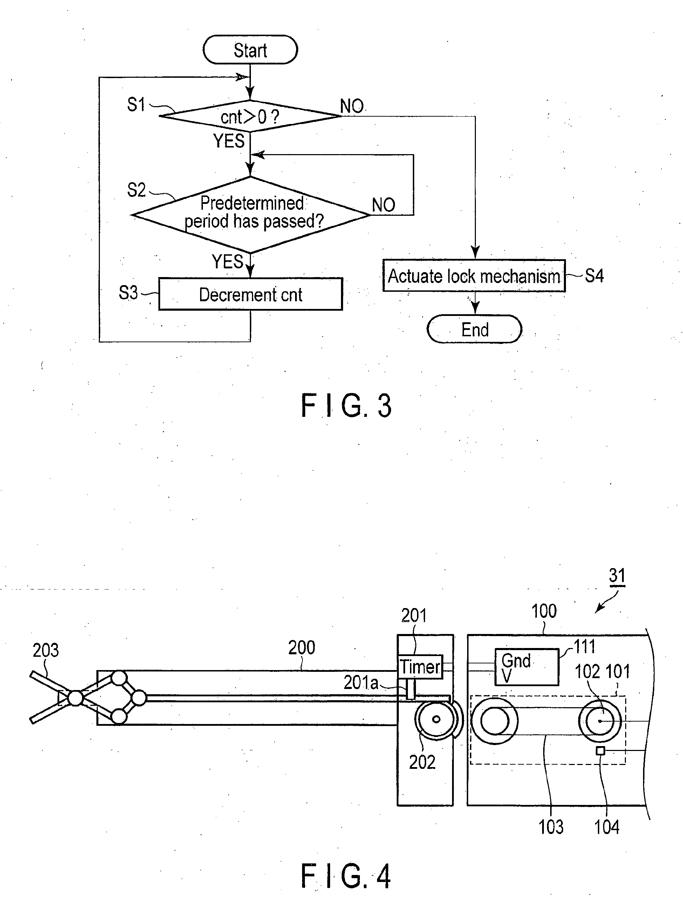

The timer 201 as an example of a use measurement unit is a timer for performing a countdown when supplied with electricity from the electricity supply unit 111. The time counted by the timer 201 corresponds to the available time (life) of the surgical instrument distal end 200. The timer 201 may be an analog timer that uses a mechanical structure such as a spring for counting, or may be a digital timer such as a digital counter. As shown in FIG. 2A, the timer 201 according to the present embodiments is provided with a lock mechanism 201a. The lock mechanism 201a is actuated and locks the operation of the power transmission unit 202 when the timer 201 counts "0", that is, when the life of the surgical instrument distal end 200 has expired. The lock mechanism 201a may use, for example, a lock member to mechanically lock the operation of the power transmission unit 202, or may use, for example, a relay to electrically lock the operation of the power transmission unit 202. For example, in the example shown in FIG. 4, the operation of the actuator unit is mechanically locked.

The power transmission unit 202 is configured to contact the power transmission unit 103 of the positioning arm 100 when the surgical instrument distal end 200 is attached to the distal end of the positioning arm 100. The power transmission unit 202 having such a configuration operates in response to the operation of the power transmission unit 103, and operates the distal movable portion 203 of the surgical instrument distal end 200. The power transmission unit 202 shown in the example of FIG. 2A operates the distal movable portion 203 by a rack-and-pinion mechanism which operates in association with the rotation of the gear of the power transmission unit 103. Here, in the example of FIG. 2A, teeth of a pinion gear are not circumferentially formed, and are formed in a part corresponding to the movable range of the distal movable portion 203.

The distal movable portion 203 is provided at the distal end of the surgical instrument distal end 200, and operates in response to the operation of the power transmission unit 202. In the example shown in FIG. 2A, a gripper which opens and shuts in response to the back-and-forth motion of a rack that constitutes the power transmission unit 202 is provided as the distal movable portion 203.

In such a configuration, when the positioning arm 100 and the surgical instrument distal end 200 are separated from each other as shown in FIG. 2A, the gear of the power transmission unit 103 is not engaged with the pinion gear of the power transmission unit 202, so that the distal movable portion 203 does not operate. The timer 201 does not operate either because electricity is not supplied to the timer 201 from the electricity supply unit 111.

On the other hand, when the surgical instrument distal end 200 is attached to the positioning arm 100 as shown in FIG. 2B, the gear of the power transmission unit 103 is engaged with the pinion gear of the power transmission unit 202, and the distal movable portion 203 operates in accordance with the input generated in the actuator unit 102. For example, in the example shown in FIG. 2B, when the motor that constitutes the actuator unit 102 is rotated in a direction A, the pinion gear of the power transmission unit 202 is rotated in a direction B accordingly. In accordance with the rotation of the pinion gear in the direction B, the rack of the power transmission unit 202 moves in a direction C to pull the distal movable portion 203. As a result, the distal movable portion 203 is shut as indicated by a direction D. When the motor that constitutes the actuator unit 102 is rotated in the opposite direction, the distal movable portion 203 is opened.

Furthermore, when the surgical instrument distal end 200 is attached to the positioning arm 100 to use the surgical instrument as shown in FIG. 2B, electricity is supplied to the timer 201 by the electricity supply unit 111, and the timer 201 starts a countdown. FIG. 3 is a flowchart showing the operation of the timer 201. The operation shown in FIG. 3 is performed while electricity is being supplied to the timer 201 by the electricity supply unit 111. When the supply of the electricity to the timer 201 from the electricity supply unit 111 is stopped, the operation shown in FIG. 3 is ended.

A predetermined number of use is set for a count cnt as an initial value. First, whether the count cnt of the timer 201 is beyond 0 is determined (step S1). When the count cnt is beyond 0 in step S1, that is, when the surgical instrument distal end 200 still has a remaining life, whether a predetermined period has passed is determined (step S2). When the predetermined period has passed in S2, the count cnt is decremented (countdown) (step S3). The processing then returns to step S1. That is, the countdown is continued until the count cnt reaches 0.

When the count cnt is 0 in step S1, that is, when the surgical instrument distal end 200 has no remaining life, the operation of the surgical instrument distal end 200 is locked by the lock mechanism 201a, for example, as shown in FIG. 4 (step S4).

Here, the operation shown in FIG. 3 includes judging whether the count cnt is beyond 0. For an analog timer that uses a mechanical structure such as a spring for counting, there is no such a judgment, and the locking operation in step S4 is performed when the count cnt has reached 0.

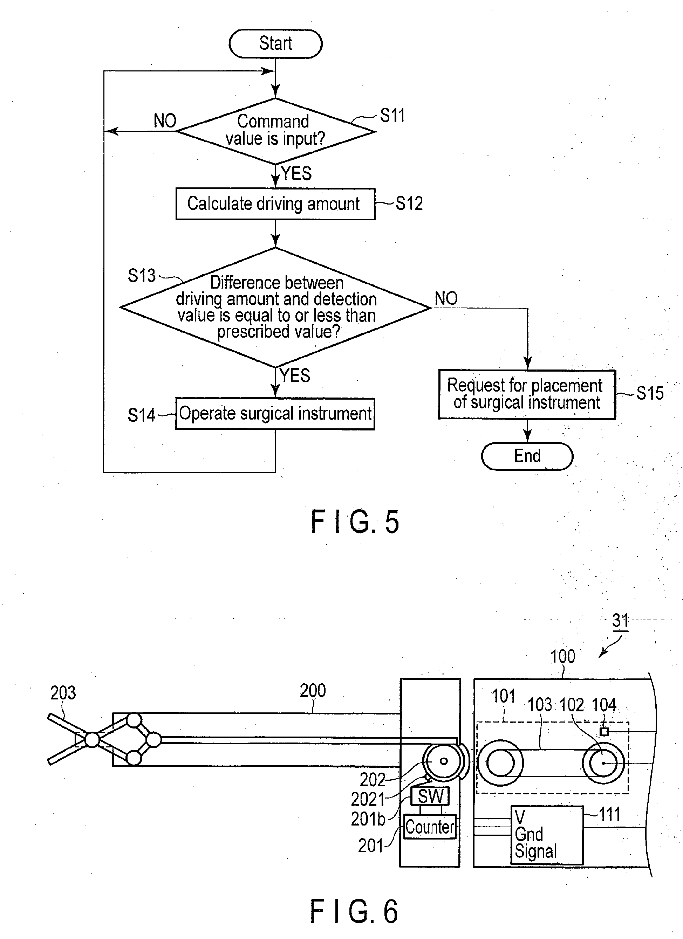

FIG. 5 is a flowchart showing the operation of the manipulator control unit 22. The manipulator control unit 22 determines whether a command value conforming to the operation of the operation unit 11 by the operator 1 is input from the master control unit 21 (step S11). When it is determined in step S11 that no command value is input, the manipulator control unit 22 performs the judgment in step S11 and waits until the command value is input. When it is determined in step S11 that a command value is input, the manipulator control unit 22 calculates, for example, by inverse-kinematic computation, a desired driving amount of the actuator unit 102 to drive each joint of the positioning arm 100 in accordance with an input command value (step S12). For the inverse-kinematic computation, various known methods such as an analytical technique can be used. The details are not described here.

After calculating the driving amount, the manipulator control unit 22 determines whether the difference between the calculated driving amount and the driving amount detecting unit 104 is equal to or less than a predetermined value (step S13). When the surgical instrument distal end 200 has no remaining life as shown in FIG. 4, the lock mechanism 201a is actuated, and the operation of the surgical instrument distal end 200 is locked. In this case, the operation of the actuator unit 102 is also locked. Therefore, the detection value of the driving amount detecting unit 104 does not change. In contrast, the driving amount of the actuator unit 102 calculated in step S12 constantly changes depending on the operation of the operation unit 11 by the operator 1. Thus, the difference between the driving amount of the actuator unit 102 and the detection value of the driving amount detecting unit 104 can be obtained to determine whether the operation of the actuator unit 102 is stopped, that is, whether the surgical instrument distal end 200 has a remaining life.

When it is determined in step S13 that the difference between the calculated desired driving amount and the detection value of the driving amount detecting unit 104 is equal to or less than the predetermined value, this means that the actuator unit 102 operates in accordance with the driving amount calculated in the manipulator control unit 22, in other words, the surgical instrument distal end 200 has a remaining life. In this case, the manipulator control unit 22 inputs the calculated desired driving amount to the actuator unit 102 of the positioning arm 100 to operate the surgical instrument distal end 200 (step S14). On the other hand, when the difference between the calculated driving amount and the detection value of the driving amount detecting unit 104 is beyond the predetermined value, this means that the operation of the surgical instrument distal end 200 is locked, in other words, the surgical instrument distal end 200 has no remaining life. In this case, the manipulator control unit 22 sends an instruction to the image processing unit 23 to display, on the display unit 12, an alarm display for encouraging the replacement of the surgical instrument distal end 200 (step S15). The manipulator control unit 22 then ends the operation shown in FIG. 5. Although the alarm display is shown here by way of example, the alarm display is not the exclusive means for encouraging the replacement of the surgical instrument distal end 200.

As described above, according to the first embodiment, the surgical instrument distal end 200 has the timer 201 therein. While the surgical instrument distal end 200 is attached to the positioning arm 100 and electricity is being supplied to the timer 201, the timer 201 performs a countdown. When the timer 201 counts 0, the operation of the surgical instrument distal end 200 is locked. Thus, the time in which the surgical instrument distal end 200 is attached to the positioning arm 100 is correctly measured in the surgical instrument distal end 200 as the life of the surgical instrument distal end 200. When the surgical instrument distal end 200 has come to the end of its life, the operation of the surgical instrument distal end 200 can be instantaneously locked.

The manipulator control unit 22 can determine by the detection value from the driving amount detecting unit 104 whether the surgical instrument distal end 200 has a remaining life. In accordance with this determination, the manipulator control unit 22 can give an alarm for encouraging the replacement of the surgical instrument distal end 200 to the operator 1. Thus, the surgical instrument distal end 200 and the manipulator control unit 22 do not need to be conductively connected.

Here, in the example described above, the timer 201 performs a countdown while electricity is being supplied to the timer 201. Otherwise, the timer 201 may perform a countdown when the surgical instrument distal end 200 is attached to the positioning arm 100. In this case, the number of times the surgical instrument distal end 200 is attached to the positioning arm 100 can be measured as the life of the surgical instrument distal end 200.

### [Second Embodiment]

Now, the second embodiment of the present invention is described. In the first embodiment described above, the time in which the surgical instrument distal end 200 is attached to the positioning arm 100 is measured as the life of the surgical instrument distal end 200. In contrast, in the example according to the second embodiment, the number of times or the time in which the surgical instrument distal end 200 is actually used (operated) is measured as the life of the surgical instrument distal end 200.

FIG. 6 is a diagram showing the configuration of a slave arm 31 according to the second embodiment of the present invention. The slave arm 31 shown in FIG. 6 also comprises a positioning arm 100 and a surgical instrument distal end 200. Components similar to those in FIG. 2A are provided with the same reference signs similar to those in FIG. 2A and are not described below.

An electricity supply unit 111 in FIG. 6 is conductively connected to a counter IC 201 in the surgical instrument distal end 200 when the surgical instrument distal end 200 is attached to the positioning arm 100. The electricity supply unit 111 having such a configuration supplies electricity for driving the counter IC 201. As shown in FIG. 6, the electricity supply unit 111 according to the present embodiment is provided with a terminal (signal) for receiving a surgical instrument replacement request signal from the counter IC 201, in addition to terminals (V, Gnd) for supplying electricity to drive the counter IC 201. The surgical instrument replacement request signal will be described later.

In the same manner as the power transmission unit 202 according to the first embodiment shown in FIG. 2A, the power transmission unit 202 shown by way of example in the second embodiment operates a distal movable portion 203 by a rack-and-pinion mechanism. In addition, in the second embodiment, a protrusion 2021 is formed in a toothless part of a pinion gear that constitutes the power transmission unit 202. A switch (SW) 201b is further provided in the vicinity of the protrusion 2021. The SW 201b is turned on by being depressed when the protrusion 2021 formed in the pinion gear comes into contact with the SW 201b by the rotation of the pinion gear that constitutes the power transmission unit 202. The SW 201b is turned off when the protrusion 2021 formed in the pinion gear comes out of contact with the SW 201b by the rotation of the pinion gear that constitutes the power transmission unit 202. When the SW 201b is turned on, a count signal indicating that the SW 201b is turned on is input to the counter IC 201 from the SW 201b.

The counter IC 201 as an example of a use measurement unit is an IC circuit for, for example, counting the count signals to measure the number of times the SW 201b is depressed. In this way, the number of times the power transmission unit 202 is operated, that is, the number of times the surgical instrument distal end 200 is actually used can be measured by counting the count signals. The counter IC 201 also performs processing to lock the operation of the surgical instrument distal end 200 when the number of the count signals has reached a predetermined value. This processing may include, for example, forcibly driving the pinion gear to disengage a gear of a power transmission unit 103 and the pinion gear of the power transmission unit 202 so that the toothless part of the pinion gear faces the gear of the power transmission unit 202. That is, this processing corresponds to the mechanism for locking.

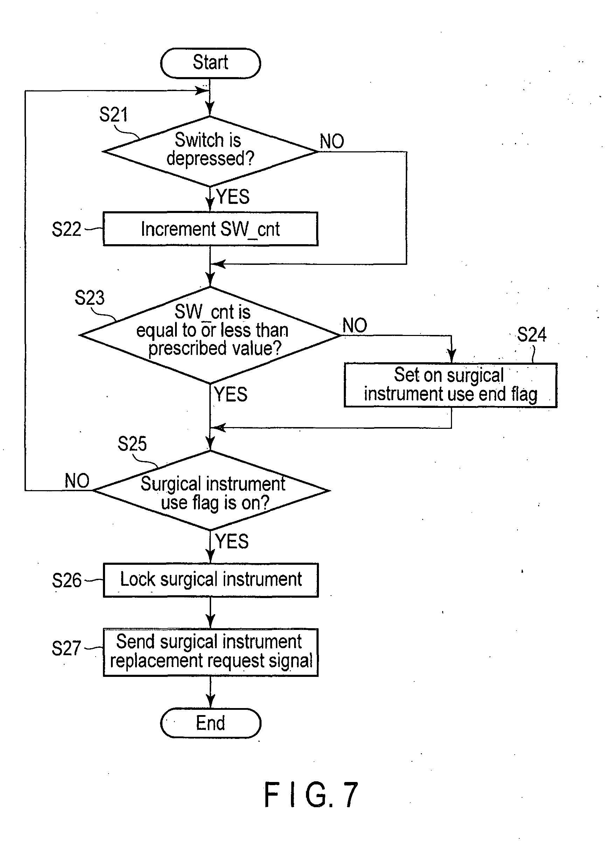

FIG. 7 is a flowchart showing the operation of the counter IC 201. As in the first embodiment, the operation shown in FIG. 7 is performed while electricity is being supplied to the counter IC 201 by the electricity supply unit 111. When the supply of the electricity to the counter IC 201 from the electricity supply unit 111 is stopped, the operation shown in FIG. 7 is ended.

First, the counter IC 201 determines by the presence of a count signal whether the SW 201b is depressed (step S21). When the SW 201b is depressed in step S21, the counter IC 201 increments (counts up) a count SW_cnt (step S22). On the other hand, when the SW 201b is not depressed in step S21, the counter IC 201 does not count up.

The counter IC 201 then compares the count SW_cnt with a predetermined value stored in a memory within the counter IC, and determines whether the count SW_cnt is equal to or less than the predetermined value as a result of the comparison (step S23). This predetermined value is the number corresponding to the life of the surgical instrument distal end 200. When the count SW_cnt is beyond the predetermined value in step S23, that is, when the number of times the SW 201b is depressed is beyond the predetermined value, the number of times the power transmission unit 202 is operated is also beyond the predetermined value. In this case, the counter IC 201 recognizes that the surgical instrument distal end 200 has no remaining life, and the counter IC 201 turns on a surgical instrument use end flag held in a register within the counter IC 201 (step S24). On the other hand, when the count SW_cnt is equal to or less than the predetermined value in step S23, the counter IC 201 recognizes that the surgical instrument distal end 200 has no remaining life, and the counter IC 201 keeps off the surgical instrument use end flag held in the register within the counter IC 201.

The counter IC 201 then determines whether the surgical instrument use end flag is on (step S25). When the surgical instrument use end flag is off in step S25, that is, when the surgical instrument still has a remaining life, the processing returns to step S21. While the surgical instrument use end flag is off, the operations in step S21 to S25 are repeated. When the surgical instrument use end flag is on in step S25, that is, when the surgical instrument has no remaining life, the counter IC 201 locks the operation of the surgical instrument distal end 200, for example, by forcibly actuating the pinion gear (step S26). The counter IC 201 then outputs a surgical instrument replacement request signal to the electricity supply unit 111 (step S27). Further, the counter IC 201 ends the operation shown in FIG. 7.

The surgical instrument replacement request signal input to the electricity supply unit 111 is input to the manipulator control unit 22 having a function as an example of a reading unit. In response to the surgical instrument replacement request signal, the manipulator control unit 22 sends an instruction to the image processing unit 23 to display, on the display unit 12, an alarm display for encouraging the replacement of the surgical instrument distal end 200, as in the processing in step S15 in FIG. 5.

As described above, according to the second embodiment, the number of times the surgical instrument distal end 200 is actually operated is measured. Therefore, as compared with the first embodiment, the life of the surgical instrument distal end 200 is more correctly measured, and the operation of the surgical instrument distal end 200 can be instantaneously locked when the surgical instrument distal end 200 has come to the end of its life.

Here, in the example described above, the number of times the surgical instrument distal end 200 is actually operated is measured as an operation amount. Otherwise, the operating time of the surgical instrument distal end 200 may be measured as an operation amount.

Although the counter IC 201 is used for measurement in the example described above, a mechanical counter may be used for measurement as in the first embodiment.

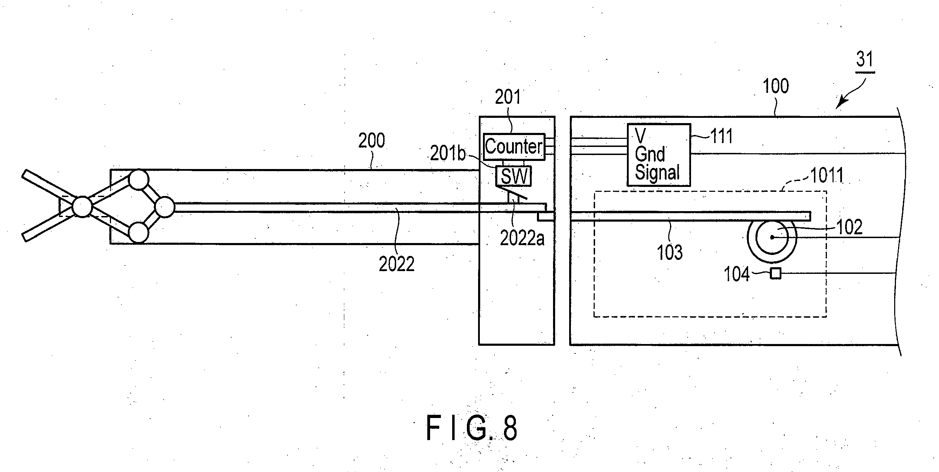

While the embodiments of the present invention have been described above, the present invention is not limited to the embodiments described above. It should be understood that various modifications and applications can be made. For example, in the examples shown in the first and second embodiments described above, the driving mechanism of the positioning arm 100 drives the surgical instrument distal end 200 by rotational input. However, the internal configuration of the positioning arm 100 is not particularly limited. For example, the driving mechanism of the positioning arm 100 may drive the surgical instrument distal end 200 by translation input. FIG. 8 shows an example of the application of the configuration according to the second embodiment in which the driving mechanism of the positioning arm 100 has a translation mechanism. A driving mechanism 1011 shown in FIG. 8 comprises a rack-and-pinion or a ball screw, and converts rotational input of a motor that constitutes the actuator unit to translation motion. In accordance with such translation motion, a rod 2022 that constitutes the power transmission unit 202 of the surgical instrument distal end 200 also performs translation motion. A protrusion 2022a similar to that described in the second embodiment is provided in the rod 2022, and an SW 201b and a counter IC 201 similar to those described in the second embodiment are provided in the vicinity of the protrusion 2022a. In such a configuration, whenever the protrusion 2022a comes into contact with the SW 201b by the translation motion of the rod 2022, the SW 201b is turned on, and the counter IC 201 counts up accordingly. This enables a life measurement as in the second embodiment. While the driving mechanism of the positioning arm 100 has the translation mechanism in the application of the configuration according to the second embodiment shown here in FIG. 8, the configuration according to the first embodiment may be applied instead.

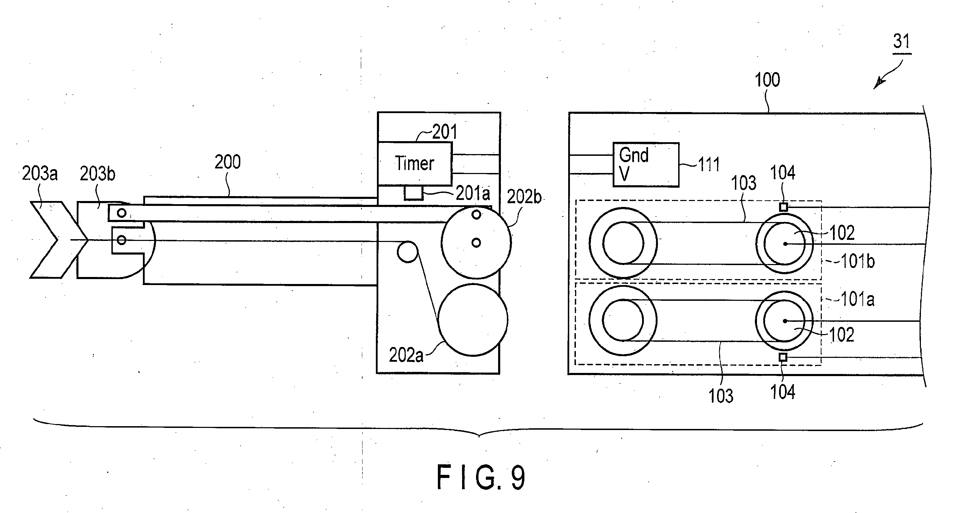

In the examples shown in the first and second embodiments described above, the distal movable portion 203 of the surgical instrument distal end 200 comprises one movable portion (gripper). Otherwise, as shown in FIG. 9, the configurations according to the embodiments described above are also applicable when the distal movable portion of the surgical instrument distal end 200 comprises multiple movable portions. In FIG. 9, the distal movable portion comprises a gripper 203a and a joint 203b. The gripper 203a and the joint 203b are then driven by different power transmission units 202a and 202b via driving mechanisms 101a and 101b, respectively. For example, in the example shown in FIG. 9, the power transmission unit 202a drives the gripper 203a by pushing/drawing operation of a wire using a pulley, and the power transmission unit 202b drives the joint 203b by a rotational input transmission mechanism having a configuration substantially similar to those in the first and second embodiments.

Furthermore, in FIG. 9, a timer 201 similar in configuration to that described in the first embodiment is provided in the vicinity of the power transmission unit 202b. In the same manner as described in the first embodiment, this timer 201 performs a countdown while the surgical instrument distal end 200 is attached to the positioning arm 100. The timer 201 locks the operation of the surgical instrument distal end 200 when the timer 201 counts 0. This enables a life measurement as in the first embodiment. Although the operation of the joint 203b is locked by the lock mechanism 201a in FIG. 9, the operation of the gripper 203a may be locked instead, or the operations of both the gripper 203a and the joint 203b may be locked. Although the application of the configuration according to the first embodiment is shown in FIG. 9, the configuration according to the second embodiment may be applied instead.

An adapter capable of transmitting input may intervene between the surgical instrument distal end 200 and the positioning arm 100.

The surgical instrument distal end 200 is attached to and detached from the positioning arm 100 at the distal end in the embodiments described above, but is not attached and detached exclusively at the distal end. The surgical instrument distal end 200 may be attached and detached at any position.

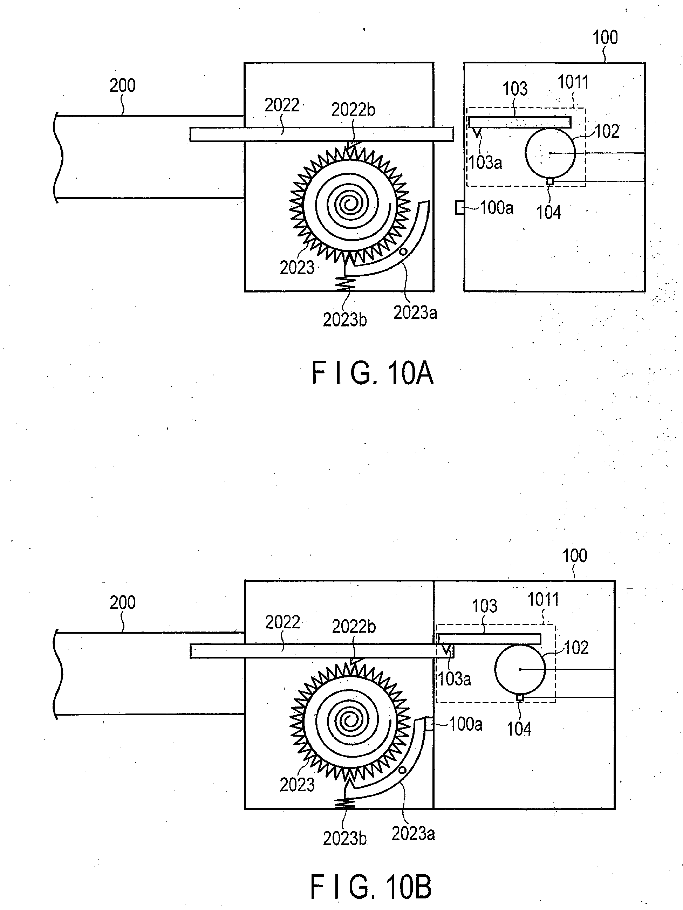

In the embodiments described above, the life of the surgical instrument distal end 200 is counted when electricity is supplied to the surgical instrument distal end 200 by the positioning arm 100. Otherwise, the attachment of the positioning arm 100 may be mechanically detected, and the operation of the surgical instrument distal end 200 may be automatically locked when the surgical instrument distal end 200 has come to the end of its life. FIG. 10A and FIG. 10B are views showing a configuration according to such a modification. The operation is the same as the operation in the flowchart of FIG. 5 described above, and is therefore not described. Although the driving mechanism for the positioning arm 100 is a translation mechanism in the example shown in FIG. 10A and FIG. 10B, the driving mechanism does not necessarily need to be the translation mechanism.

A power transmission unit 103 of the driving mechanism 1011 shown in FIG. 10A comprises, for example, a rack-and-pinion or a ball screw, and converts rotational input of a motor that constitutes an actuator unit 102 to translation motion. An engaging protrusion 103a is formed at the distal end of the power transmission unit 103. This engaging protrusion 103a is configured to engage with the rod 2022 of the surgical instrument distal end 200 as shown in FIG. 10B when the surgical instrument distal end 200 is attached to the positioning arm 100. In such a configuration, the rod 2022 also performs translation motion in accordance with the translation motion of the power transmission unit 103.

As shown in FIG. 10A, a press protrusion 100a is formed in the surface of the positioning arm 100 that faces the surgical instrument distal end 200. This press protrusion 100a is configured to come into contact with a movable portion 2023a of the surgical instrument distal end 200 so that the movable portion 2023a can turn as shown in FIG. 10B when the surgical instrument distal end 200 is attached to the positioning arm 100.

That is, as shown in FIG. 10A, the movable portion 2023a is formed to extend from the surface of the surgical instrument distal end 200 facing the positioning arm 100 to the lower part of a spring gear 2023. The movable portion 2023a locks the spring gear 2023 as shown in FIG. 10A when the surgical instrument distal end 200 is not attached to the positioning arm 100. On the other hand, when the surgical instrument distal end 200 is attached to the positioning arm 100, the movable portion 2023a turns and unlocks the spring gear as shown in FIG. 10B. A spring 2023b is provided under the movable portion 2023a. The spring 2023b is provided to return the movable portion 2023a to the state shown in FIG. 10A when the surgical instrument distal end 200 is detached from the positioning arm 100.

Even when the spring gear 2023 is unlocked by the movable portion 2023a, an unshown protrusion does not free the spring gear 2023 from the turning force produced by a spring, and the spring gear 2023 can rotate counterclockwise in the drawing. However, the spring gear 2023 is held from rotating clockwise, and is structured to save energy in the spring by rotating counterclockwise.

A protrusion 2022b is formed in the rod 2022 of the surgical instrument distal end 200. This protrusion 2022b is configured to engage with the spring gear 2023 only when the rod 2022 is moved leftward in the drawing, and not to engage with the spring gear 2023 when the rod 2022 is moved rightward in the drawing. Although not shown in FIG. 10A, the rod 2022 is the rod 2022 shown in FIG. 8 or is a driving rod for driving the gripper and joint of the surgical instrument distal end as shown in FIG. 9.

In the configuration shown in FIG. 10A, when the surgical instrument distal end 200 is attached to the positioning arm 100, the movable portion 2023a turns so that the spring gear 2023 is movable. If the rod 2022 performs translation motion in this condition, the spring gear 2023 is rotated, and energy is saved in the spring. Therefore, more energy is saved if the surgical instrument distal end 200 is moved more. Thus, when the number of times the surgical instrument distal end 200 is operated has exceeded a predetermined number and the energy saved in the spring has surpassed the input of the actuator unit 102, the surgical instrument distal end 200 cannot be driven any more (the operation of the surgical instrument distal end 200 is locked). This is the point where the surgical instrument distal end 200 reaches the end of its life. The time of the use of each surgical instrument can be set by changing the strength of the spring. The use of such a configuration also provides advantageous effects similar to those according to the first embodiment.

That is, the time in which the surgical instrument is actually used can be measured without the supply of electricity to the surgical instrument. The surgical instrument can be deactivated when the set time of use is reached. The total operation amount of the surgical instrument can be held without using, for example, an IC.

In the embodiments (including the modification) described above, the driving mechanisms 101, 1011, 101a and, 101b for driving the surgical instrument distal end 200 as a surgical instrument are provided in the positioning arm 100 which the surgical instrument distal end 200 is attached to or detached from. However, the following surgical instruments may be provided instead. One surgical instrument comprises a surgical instrument distal end 200 and an operation unit for operating the surgical instrument distal end 200. The surgical instrument distal end 200 is attachable to and detachable from the operation unit. A driving mechanism 101, 1011, 101a or, 101b is provided not in a positioning arm 100 but in the operation unit. Another surgical instrument comprises a surgical instrument distal end 200 and the above-mentioned operation unit that are combined together. In each of these surgical instruments, the driving mechanism as a driving unit may comprise a mechanical structure instead of the above-mentioned electric structure.

The operation support system described in the example according to each of the embodiments (including the modification) comprises the surgical instrument distal end 200, and the positioning arm 100 which the surgical instrument distal end 200 is attached to and detached from. However, the present invention is not limited to such an operation support system. It is also possible to use an operation support system in which a surgical instrument comprising the surgical instrument distal end 200 and an operation unit provided with the driving mechanism 101, 1011, 101a or, 101b that are combined together is attached to the positioning arm 100 having no driving unit.

Furthermore, the embodiments described above include various stages of inventions, and various inventions can be extracted by properly combining the disclosed features. For example, when the above-mentioned problems can be solved and the above-mentioned advantageous effects can be obtained even if some of all the features shown in the embodiment are eliminated, a configuration in which those features are eliminated can also be extracted as an invention.

## Claims

1. An operation support system comprising a surgical instrument, the surgical instrument comprising:
a surgical instrument distal end (200);
a positioning arm (100) having an attachment portion at its distal end, the attachment portion being configured to attach the surgical instrument distal end (200);
a driving unit (101) provided in the attachment portion and comprising an actuator unit (102), the actuator unit (102) being configured to drive the surgical instrument distal end (200); and
a control unit (20) configured to calculate a desired driving amount of the actuator unit (102) and drive the actuator unit (102) in accordance with the calculated desired driving amount; and
a use measurement unit (201) provided in the surgical instrument distal end (200) and configured to acquire a time in which the surgical instrument distal end (200) is attached to the positioning arm (100), a number of times the surgical instrument distal end (200) is operated or the operating time of the surgical instrument distal end (200) in response to the use or operation of the surgical instrument, wherein the surgical instrument distal end (200) and the driving unit (101) are attachable to or detachable from each other,
**characterized in that** the surgical instrument further comprises
a mechanism (201a, 201b) configured to lock the operation of the surgical instrument when the number of times the surgical instrument distal end (200) is operated or the operating time of the surgical instrument distal end (200) has exceeded a predetermined value;
and **in that** the operation support system further comprises
a driving amount detecting unit (104) configured to detect an actual driving amount of the actuator unit (102), wherein
the control unit (20) is further configured to determine whether the operation of the surgical instrument is locked from the difference between the calculated desired driving amount and the actual driving amount of the actuator unit (102), and give an alarm to encourage replacement of the surgical instrument when the operation of the surgical instrument is determined to be locked.

2. The operation support system according to claim 1, wherein the surgical instrument further comprises a gripper (203a) or a joint (203b),
wherein the use measurement unit (201) is configured to acquire motion of the gripper (203a) or the joint (203b) as the number of times the surgical instrument distal end (200) is operated or the operating time of the surgical instrument distal end (200).

3. The operation support system according to any one of claims 1 and 2, wherein the surgical instrument further comprises an electricity supply unit (111) provided in the attachment portion and configured to supply electricity to the use measurement unit (201).

## Patentansprüche

1. Betriebsunterstützungssystem mit einem chirurgischen Instrument, wobei das chirurgische Instrument umfasst:
ein distales Ende (200) des chirurgischen Instruments;
einen Positionierungsarm (100) mit einem Befestigungsabschnitt an seinem distalen Ende, wobei der Befestigungsabschnitt dazu eingerichtet ist, das distale Ende (200) des chirurgischen Instruments zu befestigen;
eine Antriebseinheit (101), die in dem Befestigungsabschnitt vorgesehen ist und eine Aktuatoreinheit (102) umfasst, wobei die Aktuatoreinheit (102) dazu eingerichtet ist, das distale Ende (200) des chirurgischen Instruments anzutreiben;
eine Steuereinheit (20), die dazu eingerichtet ist, einen gewünschten Antriebsbetrag der Aktuatoreinheit (102) zu berechnen und die Aktuatoreinheit (102) entsprechend dem berechneten gewünschten Antriebsbetrag anzutreiben; und
eine Verwendungsmesseinheit (201), die in dem distalen Ende (200) des chirurgischen Instruments vorgesehen und dazu eingerichtet ist, eine Zeit, während der das distale Ende (200) an dem Positionierungsarm (100) befestigt ist, eine Anzahl von Betätigungen des distalen Endes (200) des chirurgischen Instruments oder die Betriebszeit des distalen Endes (200) des chirurgischen Instruments in Reaktion auf die Verwendung oder den Betrieb des chirurgischen Instruments zu messen, wobei das distale Ende (200) des chirurgischen Instruments und die Antriebseinheit (101) aneinander befestigbar oder voneinander lösbar sind,
**dadurch gekennzeichnet, dass** das chirurgische Instrument ferner
einen Mechanismus (201a, 201b) umfasst, der dazu eingerichtet ist, den Betrieb des chirurgischen Instruments zu sperren, wenn die Anzahl von Betätigungen des distalen Endes (200) des chirurgischen Instruments oder die Betriebszeit des distalen Endes (200) des chirurgischen Instruments einen vorbestimmten Wert überschritten hat;
und dass das Betriebsunterstützungssystem ferner
eine Antriebsbetragserfassungseinheit (104) umfasst, die dazu eingerichtet ist, einen tatsächlichen Antriebsbetrag der Aktuatoreinheit (102) zu erfassen, wobei
die Steuereinheit (20) ferner dazu eingerichtet ist, zu bestimmen, ob der Betrieb des chirurgischen Instruments aufgrund der Differenz zwischen dem berechneten gewünschten Antriebsbetrag und dem tatsächlichen Antriebsbetrag der Aktuatoreinheit (102) gesperrt ist und einen Alarm auszugeben, um dazu anzuregen, das chirurgische Instrument zu ersetzen, wenn bestimmt wurde, dass der Betrieb des chirurgischen Instruments gesperrt ist.

2. Betriebsunterstützungssystem gemäß Anspruch 1, bei dem das chirurgische Instrument ferner einen Greifer (203a) oder ein Gelenk (203b) umfasst,
wobei die Verwendungsmesseinheit (201) dazu eingerichtet ist, eine Bewegung des Greifers (203a) oder des Gelenks (203b) als die Anzahl der Betätigungen des distalen Endes (200) des chirurgischen Instruments oder die Betriebszeit des distalen Endes (200) des chirurgischen Instruments zu messen.

3. Betriebsunterstützungssystem nach einem der Ansprüche 1 und 2, bei dem das chirurgische Instrument ferner eine Elektrizitätsversorgungseinheit (111) umfasst, die in dem Befestigungsabschnitt vorgesehen und dazu eingerichtet ist, der Verwendungsmesseinheit (201) Elektrizität zuzuführen.

## Revendications

1. Système de support d'actionnement comprenant un instrument chirurgical, l'instrument chirurgical comprenant :
une extrémité distale (200) d'instrument chirurgical ;
un bras de positionnement (100) comportant une partie d'attache au niveau de son extrémité distale, la partie d'attache étant configurée pour attacher l'extrémité distale (200) d'instrument chirurgical ;
une unité d'entraînement (101) prévue dans la partie d'attache et comprenant une unité d'actionneur (102), l'unité d'actionneur (102) étant configurée pour entraîner l'extrémité distale (200) d'instrument chirurgical ; et
une unité de commande (20) configurée pour calculer une quantité d'entraînement souhaitée de l'unité d'actionneur (102) et entraîner l'unité d'actionneur (102) conformément à la quantité d'entraînement souhaitée calculée ; et
une unité de mesure d'utilisation (201) prévue dans l'extrémité distale (200) d'instrument chirurgical et configurée pour acquérir un temps pendant lequel l'extrémité distale (200) d'instrument chirurgical est attachée au bras de positionnement (100), un nombre de fois que l'extrémité distale (200) d'instrument chirurgical est actionnée ou le temps d'actionnement de l'extrémité distale (200) d'instrument chirurgical en réponse à l'utilisation ou à l'actionnement de l'instrument chirurgical, dans lequel l'extrémité distale (200) d'instrument chirurgical et l'unité d'entraînement (101) peuvent être attachées l'une à l'autre ou détachées l'une de l'autre, **caractérisé en ce que** l'instrument chirurgical comprend en outre
un mécanisme (201a, 201b) configuré pour bloquer l'actionnement de l'instrument chirurgical lorsque le nombre de fois que l'extrémité distale (200) d'instrument chirurgical est actionnée ou lorsque le temps d'actionnement de l'extrémité distale (200) d'instrument chirurgical a dépassé une valeur prédéterminée ;
et **en ce que** le système de support d'actionnement comprend en outre
une unité de détection de quantité d'entraînement (104) configurée pour détecter une quantité d'entraînement réelle de l'unité d'actionneur (102), dans lequel
l'unité de commande (20) est en outre configurée pour déterminer si l'actionnement de l'instrument chirurgical est bloqué à partir de la différence entre la quantité d'entraînement souhaitée calculée et la quantité d'entraînement réelle de l'unité d'actionneur (102), et délivrer une alerte pour encourager le remplacement de l'instrument chirurgical lorsque l'actionnement de l'instrument chirurgical est déterminé comme étant bloqué.

2. Système de support d'actionnement selon la revendication 1, dans lequel l'instrument chirurgical comprend en outre un dispositif de préhension (203a) ou une articulation (203b),
dans lequel l'unité de mesure d'utilisation (201) est configurée pour acquérir les mouvements du dispositif de préhension (203a) ou de l'articulation (203b) comme le nombre de fois que l'extrémité distale (200) d'instrument chirurgical est actionnée ou le temps d'actionnement de l'extrémité distale (200) d'instrument chirurgical.

3. Système de support d'actionnement selon l'une quelconque des revendications 1 et 2, dans lequel l'instrument chirurgical comprend en outre une unité d'alimentation en électricité (111) prévue dans la partie d'attache et configurée pour délivrer l'électricité vers l'unité de mesure d'utilisation (201).
